(19) Europäisches Patentamt
European Patent Office
Office européen des brevets

(11) Veröffentlichungsnummer: **0 213 062 B1**

(12) **EUROPÄISCHE PATENTSCHRIFT**

(45) Veröffentlichungstag der Patentschrift: **31.10.90**

(51) Int. Cl.[5]: **C07D 457/12**, A61K 31/48

(21) Anmeldenummer: **86730119.4**

(22) Anmeldetag: **25.07.86**

(54) **1-Alkyl-ergolinyl-thioharnstoffderivate.**

(30) Priorität: **06.08.85 DE 3528584**

(43) Veröffentlichungstag der Anmeldung: **04.03.87 Patentblatt 87/10**

(45) Bekanntmachung des Hinweises auf die Patenterteilung: **31.10.90 Patentblatt 90/44**

(84) Benannte Vertragsstaaten: **AT BE CH DE FR GB IT LI LU NL SE**

(56) Entgegenhaltungen:
**EP-A- 0 082 808**
**AT-B- 278 853**
**DE-B- 1 119 843**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Biere, Helmut, Dr., Zeltinger Strasse 15, D-1000 Berlin 28(DE)**
Erfinder: **Sauer, Gerhard, Dr., Königsbacher Zeile 41A, D-1000 Berlin 28(DE)**
Erfinder: **Wachtel, Helmut, Dr., Asternplatz 2, D-1000 Berlin 45(DE)**

**EP 0 213 062 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue 1-Alkyl-ergolinyl-thioharnstoffderivate, ihre Herstellung und ihre Verwendung in Arzneimitteln.

Die erfindungsgemäßen Verbindungen haben die allgemeine Formel I

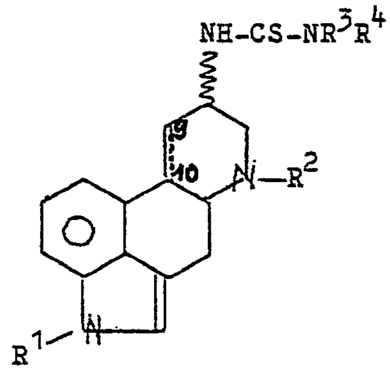

(I)

worin
$R^1$ $C_{1-4}$-Alkyl,
$R^2$ $C_{1-4}$-Alkyl,
$R^3$ und $R^4$ $C_{1-2}$-Alkyl bedeuten und
$C_9 \overset{---}{} C_{10}$ eine CC-Einfachbindung oder eine CC-Doppelbindung darstellt,
wobei, falls $R^3$ und $R^4$ Ethyl bedeuten, $R^1$ und $R^2$ nicht Methyl sein können sowie deren Säurenadditionssalze.

Der Alkylrest $R^1$ mit bis zu 4 Kohlenstoffatomen leitet sich von aliphatischen, geradkettigen verzweigten oder cyclischen Kohlenwasserstoffresten ab wie z.B. Methyl, Ethyl, n-Propyl, Isopropyl, Cyclopropyl, n-Butyl, tert.-Butyl, Isobutyl, sek.Butyl, Cyclobutyl.

Als Alkylreste $R^2$, $R^3$ und $R^4$ seien beispielsweise genannt Methyl, Ethyl, Isopropyl, n-Propyl, Butyl, Isobutyl und tert.-Butyl, sek.-Butyl.

Bedeutet $C_9 \overset{---}{} C_{10}$ eine Einfachbindung, so ist das Wasserstoffatom in 10-Stellung-α-ständig. Der Substituent in 8-Stellung kann α- oder β-ständig sein.

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkandicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Capronat, Heptanoat, Propiolat, Malonat, Succinat, Suberat, Sebacat, Fumarat, Maleat, Mandelat, Butin-1.4-dioat, Eexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycollat, Malat, Tartrat, Methansulfonat, Propansulfonat, Naphthalin-1-sulfonat oder Naphtalin-2-sulfonat.

Strukturähnliche Verbindungen werden in EP-A 82 808, EP-A 126 968 und EP-A 160 842 beschrieben.

Die neuen Verbindungen der allgemeinen Formel I zeigen im Vergleich zu den in 1-Stellung nicht alkylierten Ergolin-harnstoffderivaten und Ergolin-thioharnstoffderivaten und gegenüber den aus EP-A 82 808 bekannten 1-Methyl-ergolinyl-thioharnstoffderivaten besonders ausgeprägte zentrale $\alpha_2$-Rezeptorblockierende Wirkung. Substanzen mit einem solche Wirkprofil sind besonders wertvoll zur Behandlung von psychischen Störungen des depressiven Formkreises, da nach zentraler $\alpha_2$-Rezeptorblockade eine vermehrte Noradrenalin-Freisetzung im Gehirn bewirkt wird mit der Folge eines antidepressiven Therapieeffektes.

Die zentrale $\alpha_2$-Rezeptorblockade der der erfindungsgemäßen Verbindungen wurde in einem Interaktionstest mit dem $\alpha_2$-Rezeptoragonisten Clonidin an Mäusen nach einmaliger i.p-Vorbehandlung dargestellt (Parameter: Aufhebung der durch Clonidin 0,1 mg/kg i.p. verursachten Hypothermie). Männliche NMRI-Mäuse wurden mit verschiedenen Dosen von 1-alkylierten 8-Ergolin-thioharnstoffen, die selbst nicht die Thermoregulation der Versuchstiere beeinflußen, bzw. mit Trägermedium vorbehandelt. 30 Minuten später erhielten alle Tiere Clonidin 0,1 mg/kg i.p. 60 Minuten nach Gabe von Prüfsubstanz bzw.

Trägermedium (= 30 Minuten nach Clonidin) wurde die Rektaltemperatur mit Hilfe einer Thermosonde gemessen. Während die mit Trägermedium vorbehandelten Mäuse eine Hypothermie aufwiesen, war an mit 1-substituierten Ergolin-thioharnstoffen vorbehandelten Tieren der körpertemperatursenkende Effekt des Clonidin dosisabhängig aufgehoben.

Die Herstellung der Verbindungen der allgemeinen Formel I erfolgt nach an sich bekannten Verfahren, beispielsweise indem man

a) ein Ergolin-harnstoffderivat der allgemeinen Formel II

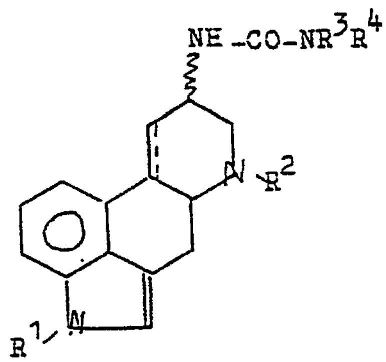

(II)

worin $R^1$, $R^2$, $R^3$, $R^4$ und C- - -C die oben genannte Bedeutung haben, mit $POCl_3$ und Alkali-Xanthogenat umsetzt oder

b) ein Amin der allgemeinen Formel III

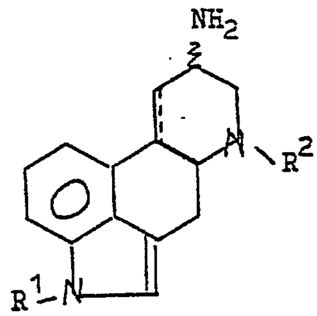

(III)

worin $R^1$, $R^2$ und C- - -C die obige Bedeutung haben, mit 1,1′-Thiocarbonyldiimidazol und einem Amin der allgemeinen Formel

$$HNR^3R^4$$

worin $R^3$ und $R^4$ die obige Bedeutung haben, umsetzt und gegebenenfalls anschließend die Säureadditionssalze bildet.

Die Überführung der Harnstoffderivate in die entsprechenden Thione nach Verfahrensvariante a) erfolgt durch Umsetzung mit einem Chlorierungsmittel wie z.B. Phosphoroxychlorid und nachfolgender Reaktion mit Schwefel-Nucleophilen wie z.B. Alkalixanthogenaten.

Die Reaktion wird in inerten Lösungsmitteln beispielsweise Ethern wie Diethylether, Diisopropylether, chlorierten Kohlenwasserstoffen wie Dichlormethan, Dichlorethan, Ketonen, Acetonitril u.a. bei –20°C Raumtemperatur vorgenommen.

Die Reaktionszeit beträgt 1–5 Stunden. Im allgemeinen wird unter Inertgasatmosphäre wie z.B. Stickstoff und Argon gearbeitet.

Bei der Umsetzung mit 1,1′-Thiocarbonyl-diimidazol nach Verfahrensvariante b) wird ein reaktives Intermediat gebildet, das ohne Isolierung mit einem sekundären Amin umgesetzt wird. Die Reaktion wird in inerten Lösungsmitteln beispielsweise Kohlenwasserstoffen wie Hexan, Toluol, halogenierten Kohlenwasserstoffen wie Methylenchlorid, Ethern wie Diethylether, Estern wie Essigsäureethylester u.a. bei Raumtemperatur bis 70°C durchgeführt und ist nach 1–3 Stunden beendet.

Zur Bildung von Salzen werden die so erhaltenen Verbindungen der allgemeinen Formel I in wenig Methanol oder Methylenchlorid gelöst und mit einer konzentrierten Lösung der gewünschten Säure in Methanol bei Raumtemperatur versetzt.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Die folgenden Beispiele sollen das erfindungsgemäße Verfahren erläutern.

Die Herstellung des Ausgangsmaterials ist entweder bekannt oder kann nach dem Fachmann bekannten Verfahren erfolgen.

Beispielsweise wird die $N^1$-Alkylierung beschrieben zur Darstellung von

1,1-Diethyl-3-(1,6-di-(n)-propyl-8α-ergolinyl)-harnstoff

2 g 1,1-Diethyl-3-(6-X-(n)-propyl-8α-ergolinyl)-harnstoff, 2,7 g gepulvertes KOH, 217 mg Tetrabutylammoniumhydrogensulfat und 5,5 ml n-Propyljodid werden in 100 ml abs. Tetrahydrofuran unter Stickstoff 5 Stunden bei Raumtemperatur gerührt. Nach Zusatz von 50 ml $H_2O$ wird mit Ethylacetat extrahiert, nachgewaschen und getrocknet. Man erhält 1,8 g (80% d.Th.) als Öl.

$$[\alpha]_D = + 2{,}6° \ (c = 0{,}5 \ CHCl_3)$$

Beispiel 1

1,1-Diethyl-3-(1-ethyl-9,10-didehydro-6-methyl-8α-ergolinyl)-thioharnstoff

Eine Lösung von 0,85 g Phosphoroxychlorid in 6 ml Dichlormethan wird auf −20°C abgekühlt und dann unter Stickstoff mit 660 mg 1,1-Diethyl-3-(1-ethyl-9,10-didehydro-6-methyl-8α-ergolinyl)-harnstoff versetzt. Das Reaktionsgemisch wird 5 Stunden bei −20°C, dann über Nacht bei Raumtemperatur gerührt. Nach dem Abdestillieren von Dichlormethan und überschüssigem $POCl_3$ wird der Rückstand unter Feuchtigkeitsausschluß mit absolutem Diethylether verrührt, gekühlt und das Kristallisat abgesaugt. Nach Aufschlämmen in 5 ml absolutem Acetonitril wird auf -10°C abgekühlt, mit 0,9 g Kaliumethylxanthogenat versetzt und unter Feuchtigkeitsausschluß zunächst 2 Stunden bei −10°C, dann weitere 4 Stunden bei Raumtemperatur gerührt. Danach wird das Acetonitril abdestilliert, der Rückstand mit $NaHCO_3$-Lösung und Ethylacetat versetzt, die organische Phase abgetrennt und der Rückstand an Kieselgel chromatographiert. Ausbeute: 480 mg (70% d.Th.); Schmelzpunkt 180°C (Dichlormethan/Pentan).
$[\alpha]_D = + 354°$ (c = 0,5, $CHCl_3$)

Beispiel 2

1,1-Diethyl-3-(1,6-di-(n)-propyl-8α-ergolinyl)-thioharnstoff

Analog Beispiel 1 aus 1,1-Diethyl-3-(1,6-di-(n)-propyl-8α-ergolinyl)-harnstoff
$[\alpha]_D = + 39°$ (c = 0,5, $CHCl_3$)

Beispiel 3

1,1-Diethyl-3-(1-ethyl-6-methyl-8α-ergolinyl)-thioharnstoff

Man löst 758 mg 1,1-Diethyl-3-(1-ethyl-6-methyl-8α-ergolinyl)-harnstoff in 75 ml 1n Salzsäure und erhitzt 8 Stunden auf 110°C. Dann wird mit konz. Ammoniumhydroxid-Lösung alkalisch gemacht und mit Methylenchlorid ausgeschüttelt. Die organische Phase wird mit Natriumsulfat getrocknet und eingedampft.

Das Rohprodukt (8α-Amino-1-ethyl-6-methyl-ergolin) wird in 30 ml wasserfreiem Methylenchlorid gelöst, mit 500 mg N,N'-Thiocarbonyldiimdazol versetzt und 1 Stunde bei Raumtemperatur gerührt. Dann gibt man 1 ml Diethylamin zu und rührt eine weitere Stunde bei Raumtemperatur. Man gibt 30 ml Wasser zu, rührt noch 30 Minuten und arbeitet dann wie oben beschrieben auf. Der Rückstand wird durch Chromatographie an Kieselgel gereinigt und kristallisiert. Ausbeute: 180 mg (17% d.Th.);
$[\alpha]_D = +28°$ (c = 0,5, Chloroform)

In analoger Weise werden dargestellt:

1,1-Diethyl-3-(6-methyl-1-n-propyl-8α-ergolinyl)-thioharnstoff
Ausbeute: 14% (kristallisiert als weinsaures Salz),
$[\alpha]_D = +37°$ (c = 0,5, Pyridin)

1,1-Diethyl-3-(6-methyl-1-isopropyl-8α-ergolinyl)-thioharnstoff
Ausbeute: 64%
$[\alpha]_D = +27°$ (c = 0,5, Chloroform)

## Patentansprüche

1. 1-Alkyl-ergolinyl-thioharnstoffderivate der allgemeinen Formel I

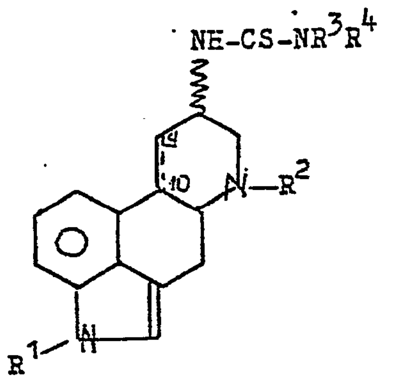

(I)

worin
$R^1$ ein $C_{1-4}$-Alkyl
$R^2$ $C_{1-4}$-Alkyl
$R^3$ und $R^4$ $C_{1-2}$-Alkyl bedeuten und
$C_9$ --- $C_{10}$ eine CC-Einfachbindung oder eine CC-Doppelbindung darstellt.
wobei, falls $R^3$ und $R^4$ Ethyl bedeuten, $R^1$ und $R^2$ nicht Methyl sein können sowie deren Säureadditionssalze.

2. 1,1-Diethyl-3-(1-ethyl-9,10-didehydro-6-methyl-8α-ergolinyl)-thioharnstoff

3. 1,1-Diethyl-3-(1-ethyl-6-methyl-8α-ergolinyl)-thioharnstoff

4. 1,1-Diethyl-3-(1,6-di-(n)-propyl-8α-ergolinyl-thioharnstoff

5. 1,1-Diethyl-3-(6-methyl-1-n-propyl)-8α-ergolinyl)-thioharnstoff

6. 1,1-Diethyl-3-(6-methyl-1-isopropyl-8α-ergolinyl)-thioharnstoff

7. Verfahren zur Herstellung der Verbindungen der allgemeinen Formel I dadurch gekennzeichnet, daß man

a) ein Ergolinyl-harnstoffderivat der allgemeinen Formel II

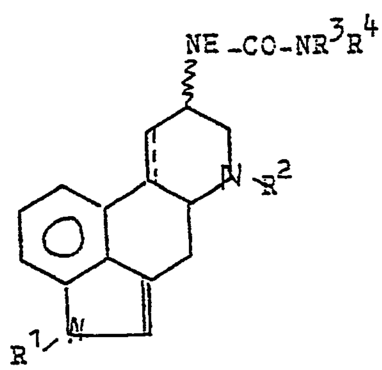

(II)

worin $R^1$, $R^2$, $R^3$, $R^4$ und C---C die oben genannte Bedeutung haben mit $POCl_3$ und Alkali-Xanthogenat umsetzt oder

b) ein Amin der allgemeinen Formel III

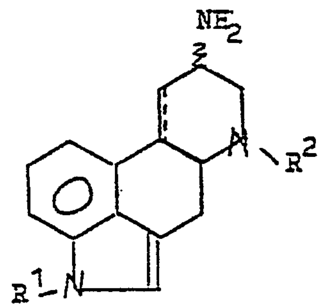

(III)

worin $R^1$, $R^2$ und C- - -C die obige Bedeutung haben, mit 1,1'-Thiocarbonyldiimidazol und einem Amin der allgemeinen Formel

$HNR^3R^4$

worin $R^3$ und $R^4$ die obige Bedeutung haben, umsetzt und gegebenenfalls die Säureadditionssalze bildet.

8. Verwendung der Verbindungen nach Anspruch 1–6 zur Herstellung eines Arzneimittels zur Behandlung von psychischen Störungen des depressiven Formenkreises.

9. Arzneimittel auf Basis der Verbindungen nach den Ansprüchen 1–6.

## Claims

1. 1-alkylergolinylthiourea derivatives of the general formula I

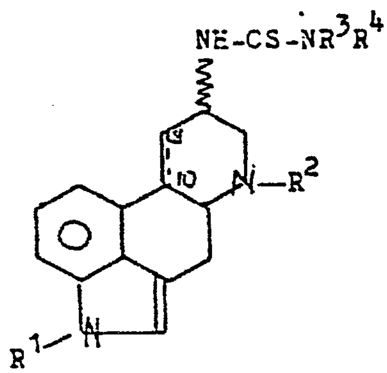

(I)

wherein
$R^1$ is $C_{1-4}$alkyl,
$R^2$ is $C_{1-4}$alkyl,
$R^3$ and $R^4$ are $C_{1-2}$alkyl and
$C_9$ - - - $C_{10}$ is a CC single bond or a CC double bond, wherein, if $R^3$ and $R^4$ are ethyl, $R^1$ and $R^2$ cannot be methyl, and the acid addition salts thereof.

2. 1,1-Diethyl-3-(1-ethyl-9,10-didehydro-6-methyl-8α-ergolinyl)thiourea.
3. 1,1-Diethyl-3-(1-ethyl-6-methyl-8α-ergolinyl)thiourea.
4. 1,1-Diethyl-3-(1,6-di-(n)-propyl-8α-ergolinyl)thiourea.
5. 1,1-Diethyl-3-(6-methyl-1-n-propyl-8α-ergolinyl)thiourea.
6. 1,1-Diethyl-3-(6-methyl-1-isopropyl-8α-ergolinyl)thiourea.
7. Process for the preparation of the compounds of the general formula I, characterised in that
a) an ergolinylthiourea derivative of the general formula II

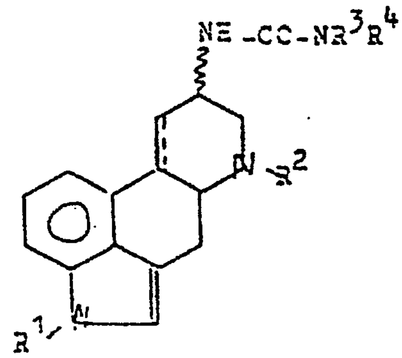

(II),

wherein $R^1$, $R^2$, $R^3$, $R^4$ and C- - -C have the meanings given above, is reacted with $POCl_3$ and alkali metal xanthogenate, or
b) an amine of the general formula III

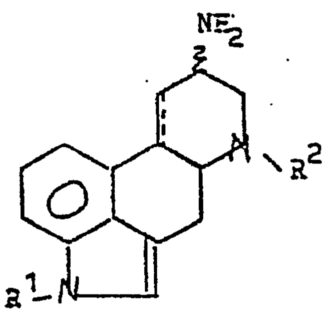

(III),

wherein $R^1$, $R^2$ and C- - -C have the meanings given above, is reacted with 1,1'-thiocarbonyldiimidazole and an amine of the general formula

$HNR^3R^4$

wherein $R^3$ and $R^4$ have the meanings given above, and, optionally, the acid addition salts are formed.

8. Use of the compounds according to claims 1 to 6 for the manufacture of a medicament for the treatment of psychic disorders of the depressive type.

9. Medicament based on the compounds according to claims 1 to 6.

**Revendications**

1. (Alkyl-1 ergolinyl)-thio-urées répondant à la formule générale I:

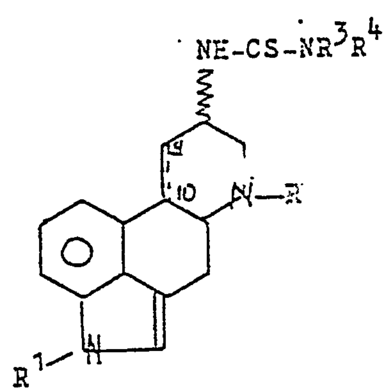

dans laquelle
$R^1$ représente un alkyle contenant de 1 à 4 atomes de carbone,
$R^2$ représente un alkyle contenant de 1 à 4 atomes de carbone,
$R^3$ et $R^4$ représentent chacun un alkyle contenant 1 ou 2 atomes de carbone,

$C_9$ --- $C_{10}$ représente une liaison carbone-carbone simple ou double et ni $R^1$ ni $R^2$ ne peuvent représenter un méthyle dans le cas où $R^3$ et $R^4$ représentent chacun un éthyle, ainsi que les sels d'addition qu'elles forment avec des acides.

2. Diéthyl-1,1 (éthyl-1 didéhydro-9,10 méthyl-6 ergolinyl-8α)-3 thio-urée.
3. Diéthyl-1,1 (éthyl-1 méthyl-6 ergolinyl-8α)-3 thio-urée.
4. Diéthyl-1,1 (di-n-propyl-1,6 ergolinyl)-8α)-3 thio-urée.
5. Diéthyl-1,1 (méthyl-6-n-propyl-1 ergolinyl-8α)-3 thio-urée.
6. Diéthyl-1,1 (méthyl-6 isopropyl-1 ergolinyl-8α)-3 thio-urée.
7. Procédé pour préparer les composés de formule générale I, procédé caractérisé en ce que:

a) on fait réagir une ergolinyl-urée répondant à la formule générale II:

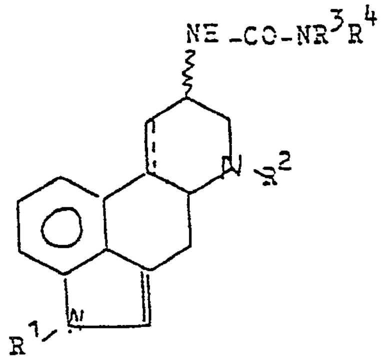

(II)

dans laquelle $R^1$, $R^2$, $R^3$, $R^4$ et $C_9$ --- $C_{10}$ ont les significations qui leur ont été données ci-dessus, avec $POCl_3$ et un xanthogénate de métal alcalin, ou

b) on fait réagir une amine répondant à la formule générale III:

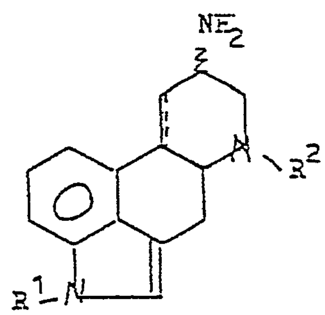

(III)

dans laquelle $R^1$, $R^2$ et $C_9$ --- $C_{10}$ ont les significations qui leur ont été données ci-dessus, avec le thiocarbonyl-1,1′ diimidazole et une amine répondant à la formule générale:

$HNR^3R^4$

dans laquelle $R^3$ et $R^4$ ont les significations qui leur ont été données ci-dessus, et éventuellement on forme les sels d'addition d'acides.

8. Application des composés selon l'une quelconque des revendications 1 à 6 à la fabrication d'un médicament destiné au traitement de troubles psychiques du syndrome dépressif.

9. Médicament à base de composés selon l'une quelconque des revendications 1 à 6.